# EUROPEAN PATENT SPECIFICATION

(11) **EP 4 043 024 B1**
(45) Date of publication and mention of the grant of the patent: **03.09.2025**
(21) Application number: 20873461.6
(22) Date of filing: 08.10.2020
(51) Int. Cl.: A61K 36/83, A61K 36/716, A61K 36/8988, A61P 25/28, A61P 25/16, A23L 33/105, A23K 10/30, A23K 40/30, A23K 50/40

(54) **COMPOSITION FOR PREVENTING OR TREATING PARKINSON'S DISEASE CONTAINING MIXED HERBAL EXTRACT OF GENKWAE FLOS, CLEMATIDIS RADIX, AND GASTRODIAE RHIZOMA**
ZUSAMMENSETZUNG ZUR VORBEUGUNG ODER BEHANDLUNG DER PARKINSON-KRANKHEIT MIT EINEM GEMISCHTEN KRÄUTEREXTRAKT AUS GENKWAE FLOS, CLEMATIDIS RADIX UND GASTRODIAE RHIZOMA
COMPOSITION DE PRÉVENTION OU DE TRAITEMENT DE LA MALADIE DE PARKINSON, CONTENANT UN EXTRAIT D'UN MÉLANGE DE PLANTES COMPOSÉ DE FLOS GENKWAE, DE RACINE DE CLÉMATITE ET DE RHIZOME DE GASTRODIA

(30) Priority: 10.10.2019 KR 20190125714
(43) Date of publication of application: 17.08.2022
(73) Proprietor: Mthera Pharma Co., Ltd., Seoul 07793 (KR)
(72) Inventor: SOHN, Miwon, Yongin-si, Gyeonggi-do 16822 (KR); CHOI, Jin Gyu, Seoul 03505 (KR); KIM, Sinyeon, Seoul 07063 (KR); PARK, Sang Cheol, Seoul 03115 (KR); KIM, Se Woong, Seoul 07522 (KR)
(74) Representative: Gassner, Birgitta
(86) International application number: PCT/KR2020/013812
(87) International publication number: WO 2021/071321

(56) References cited:
- WO-A1-2018/088862
- CN-A- 103 432 426
- CN-A- 104 547 897
- KR-A- 20110 138 531
- KR-A- 20160 058 992
- KR-B1- 101 859 196
- BAEK-SOO HAN, KYOUNG-SHIM KIM, YU JIN KIM, HOE-YUNE JUNG, YOUNG-MI KANG, KYU-SUK LEE, MI-JIN SOHN, CHUN-HYUNG KIM, KWANG-SOO KIM, : "Daphnane Diterpenes from Daphne genkwa Activate Nurr1 and Have a Neuroprotective Effect in an Animal Model of Parkinson’s Disease", JOURNAL OF NATURAL PRODUCTS, AMERICAN CHEMICAL SOCIETY, US, vol. 79, no. 6, 24 June 2016 (2016-06-24), US, pages 1604 - 1609, XP055486196, ISSN: 0163-3864, DOI: 10.1021/acs.jnatprod.6b00110

## Description

### Technical Field

The present invention relates to a pharmaceutical composition for preventing or treating neurodegenerative diseases, the composition containing a mixed herbal extract of Genkwae Flos, Clematidis Radix, and Gastrodiae Rhizoma as an active ingredient. In addition, is disclosed a food composition or feedstuff composition not claimed herein for preventing or improving neurodegenerative diseases, wherein the composition contains the herbal extract as an active ingredient. The invention is defined by the appended claims. The term "disclosure" as used herein is to be understood as "disclosure not forming part of the invention".

### Background Art

Recently, with the rapid increase of the elderly population, the number of patients with various neurodegenerative brain diseases is increasing, and interest in treatment and prevention thereof is increasing. Neurodegenerative brain disease is a disease that causes several pathologies such as motor disorders, memory disorders, and cognitive disorders due to degenerative changes in nerves.

In particular, Parkinson's disease is known as a disease caused by cell death in dopaminergic nerve containing melanin pigment in the substantia nigra pars compacta (SNpc) of the brain and dopamine deficiency in the striatum through various stimuli such as excessive production of reactive oxygen species, oxidative stress, misfolded proteins, and impaired mitochondrial function. Dopamine is produced in the substantia nigra of the brain, and the nerve cells in the substantia nigra are intricately connected to the motor cortex and other several parts of the brain, and are connected to a region called the basal ganglia that enables the body to perform movements smoothly and accurately, and dopamine regulates the function of the basal ganglia. Parkinson's disease, which is caused by a lack of dopamine secreted from the substantia nigra, is accompanied by symptoms such as tremor, bradykinesia, rigidity, postural instability, and akinesia. Until now, a method for treating Parkinson's disease have mainly used dopamine supplementation by administering L-dopa, a dopamine precursor, but it is known in many clinical studies that continuous use of L-dopa causes serious side effects (McGeer PL, McGeer EG. Glial reactions in Parkinson's disease. Mov Disord. 2008;23 (4):474-83.). Therefore, the development of natural medicines with fewer side effects than synthetic medicines in Parkinson's disease can be of great help to patients.

Therefore, in order to minimize side effects, therapeutic agents using natural substances are being developed. For example, a composition for preventing or treating Parkinson's disease containing an extract of Ampelopsis Radix as an active ingredient (Korean Patent Registration No. 10-1436017), a composition for preventing or treating Parkinson's disease containing an extract of Moutan Cortex Radicis (Korean Patent Registration No. 10-1166481) and the like are known.

On the other hand, Genkwae Flos is a flower bud of *Daphne genkwa* Siebold et Zuccarini of the family *Thymeleaceae.* Genkwae Flos acts on lung meridian, spleen meridian, and kidney meridian, and has a diuretic effect and has the efficacy of removing phlegm, and has been used as an oriental medicine to treat asthma, cough, edema, and food poisoning. Clematidis Radix is a root and a rhizome of *Clematis manshurica* Ruprecht of the family *Ranunculaceae,* and alternative related plants of the same genus, such as *Clematis hexapetala* Pallas, *Clematis chinensis* Osbeck, *Clematis trichotoma* Nakai, *Clematis armandii.* Clematidis Radix is used to eliminate rheumatism, and to treat joint flexion weakness, quadriplegia, back pain, pain in the extremities, muscle paralysis, and bruise, and is used for hyperfunction of the five viscera and pain caused by blockage of the meridian pathway. In addition, as pharmacological actions, blood pressure lowering, smooth muscle excitation, diuretic action, blood sugar lowering action, analgesic action, and antibacterial action have been reported. Gastrodiae Rhizoma is a steamed and dried tuber of *Gastrodia elata BLUME* of the family *Orchidaceae.* Gastrodiae Rhizoma acts on the liver and is used for convulsive seizure, tetanus, childhood acute and chronic convulsion, dizziness, headache, neurasthenia, headache, and the like. In addition, Gastrodiae Rhizoma exhibits the actions of sedation, anticonvulsant, analgesia, anti-inflammation, increase in cardiac and cerebral blood flow, blood pressure lowering, increase in antioxidant power, and immune activation.

However, until now, it has been not known whether a mixed herbal extract of Genkwae Flos, Clematidis Radix, and Gastrodiae Rhizoma can be effectively used for the treatment of neurodegenerative diseases such as Parkinson's disease.

Under this background, the present inventors have repeatedly studied to develop a method for treating neurodegenerative diseases. As a result, it was found that when a mixed herbal extract of Genkwae Flos, Clematidis Radix, and Gastrodiae Rhizoma was used, there was a significantly superior therapeutic effect than using each single extract. Based on the above, the present inventors completed the present invention.

### Prior Art Document

### Patent Document

(Patent Document 1) Korean Patent Registration No. 10-1436017, (Patent Document 2) Korean Patent Registration No. 10-1166481 as well as the documents CN104547897A1 and WO2018088862A1 are known.

### Detailed Description of the Invention

### Technical Problem

An object of the present invention is to provide a pharmaceutical composition for preventing or treating neurodegenerative diseases, the composition containing a mixed herbal extract of Genkwae Flos, Clematidis Radix, and/or Gastrodiae Rhizoma as an active ingredient.

Is also mentioned herein without being claimed a food composition for preventing or improving neurodegenerative diseases, the composition containing a mixed herbal extract of Genkwae Flos, Clematidis Radix, and/or Gastrodiae Rhizoma as an active ingredient.

As well, is mentioned herein without being claimed a feedstuff composition for preventing or improving neurodegenerative diseases, the composition containing a mixed herbal extract of Genkwae Flos, Clematidis Radix, and/or Gastrodiae Rhizoma as an active ingredient.

### Solution to Problem

The present invention provides a pharmaceutical composition for preventing or treating neurodegenerative diseases as claimed.

The pharmaceutical composition contains a herbal extract of Genkwae Flos and Clematidis Radix.

The pharmaceutical composition contains a herbal extract of Gastrodiae Rhizoma.

The herbal extracts are 70% ethanol extracts.

The neurodegenerative disease is Parkinson's disease.

The blending weight ratio of Genkwae Flos, Clematidis Radix, and Gastrodiae Rhizoma is 1 : 10 : 10.

In one embodiment, the pharmaceutical composition may prevent or treat neurodegenerative diseases by enhancing behavioral ability.

In another embodiment, the pharmaceutical composition may prevent or treat neurodegenerative diseases by inhibiting cell death caused by toxicity in nerve cells.

In another embodiment, the pharmaceutical composition may prevent or treat neurodegenerative diseases by inhibiting the production of reactive oxygen species (ROS) in nerve cells.

In another embodiment, the pharmaceutical composition may prevent or treat neurodegenerative diseases by protecting the function of mitochondria in nerve cells.

In another embodiment, the pharmaceutical composition may prevent or treat neurodegenerative diseases by inhibiting apoptosis of nerve cells.

In another embodiment, the pharmaceutical composition may prevent or treat neurodegenerative diseases by inhibiting the production of nitric oxide (NO).

The disclosure also mentions a feedstuff composition not claimed for preventing or improving neurodegenerative diseases, the composition containing a herbal extract of two or more selected from the group consisting of Genkwae Flos, Clematidis Radix, and Gastrodiae Rhizoma.

### Effects of the Invention

The pharmaceutical composition of the present invention containing a mixed herbal extract of Genkwae Flos, Clematidis Radix, and/or Gastrodiae Rhizoma as an active ingredient exhibits excellent therapeutic effects in neurodegenerative diseases, particularly Parkinson's disease, and can be used as a novel therapeutic agent.

In addition, the pharmaceutical composition of the present invention can be used as a safe therapeutic agent by reducing the side effects of existing therapeutic agents by including a natural product as an active ingredient.

### Brief Description of Drawings

Fig. 1 is a graph showing the results of Rotarod test in an animal model of Rotenone-induced Parkinson's disease (####: p<0.0001; ****: p<0.0001 (t-test with the Rotenone group); *: p<0.05 (t-test with the Rotenone group)).
Fig. 2 is a graph showing the cell viability when the herbal extract of the present invention is used (####: p<0.0001; ****: p<0.0001 (t-test with the MPP group); ***: p<0.001 (t-test with the MPP group)).
Fig. 3 is a graph showing the production rate of reactive oxygen species (ROS) in the cells when the herbal extract of the present invention is used (##: p<0.01; **: p<0.01 (t-test with the MPP group)).
Fig. 4 is a graph showing the ability of protecting mitochondrial function when the herbal extract of the present invention is used as a TMRE measurement rate (####: p<0.0001; *: p<0.05 (t-test with the MPP group)).
Fig. 5 is a graph showing the ability of reducing apoptosis when the herbal extract of the present invention is used (####: p<0.0001; ****: p<0.0001 (t-test with the MPP group); ***: p<0.001 (t-test with the MPP group)).
Fig. 6 is a graph showing the result of measuring the production of nitric oxide (NO) when the herbal extract of the present invention is used (####: p<0.0001; ****: p<0.0001 (t-test with the LPS group); ***: p<0.001 (t-test with the LPS group)).
Fig. 7 is a graph showing the results of Rotarod test in an animal model of Rotenone-induced Parkinson's disease (####: p<0.0001; ***: p<0.001 (t-test with the Rotenone group); **: p<0.01 (t-test with the Rotenone group)).
Fig. 8 is a graph showing the result of measuring the production of nitric oxide (NO) when the herbal extract of the present invention is used (####: p<0.0001; **: p<0.01 (t-test with the LPS group); *: p<0.05 (t-test with the LPS group)).
Figs. 9a and 9b are graphs showing the cytoprotective effect against MPP+ or 6-OHDA toxicity when the herbal extract of the present invention is used.
Fig. 10 is a graph showing the results of Pole test in an animal model of MPTP-induced Parkinson's disease.
Fig. 11 is a graph showing the results of Rotarod test in an animal model of MPTP-induced Parkinson's disease.

### Best Mode for Carrying out the Invention

Hereinafter, embodiments and examples of the present invention will be described in detail with reference to the accompanying drawings so that those of ordinary skill in the art to which the present invention pertains can easily carry out. However, the present application may be implemented in several forms and is not limited to the embodiments and examples described herein.

Throughout the present specification, when a part "includes" a component, it means that other components may be further included, rather than excluding other components, unless otherwise stated.

The present disclosure relates to a pharmaceutical composition for preventing or treating neurodegenerative diseases, the composition containing a herbal extract of two or more selected from the group consisting of Genkwae Flos, Clematidis Radix, and Gastrodiae Rhizoma.

For example, the pharmaceutical composition may contain a herbal extract of Genkwae Flos and Clematidis Radix.

For example, the pharmaceutical composition may contain a herbal extract of Genkwae Flos and Gastrodiae Rhizoma.

For example, the pharmaceutical composition may contain a herbal extract of Clematidis Radix and Gastrodiae Rhizoma.

For example, the pharmaceutical composition may contain a herbal extract of Genkwae Flos, Clematidis Radix, and Gastrodiae Rhizoma.

As used herein, the term "herbal extract" or "mixed herbal extract" refers to an active ingredient of the pharmaceutical composition of the present disclosure and may include each extract extracted from a herb selected from the group consisting of Genkwae Flos, Clematidis Radix, and Gastrodiae Rhizoma, or may include an extract extracted from a mixture of these herbs.

For example, the pharmaceutical composition contains a herbal extract of Genkwae Flos and Clematidis Radix, and the blending weight ratio of the two herbs or their herbal extracts may be 1 : 1 to 20, preferably 1 : 5 to 15, and more preferably 1 : 10.

For example, the pharmaceutical composition contains a herbal extract of Genkwae Flos and Gastrodiae Rhizoma, and the blending weight ratio of the two herbs or their herbal extracts may be 1 : 1 to 20, preferably 1 : 5 to 15, and more preferably 1 : 10.

For example, the pharmaceutical composition contains a herbal extract of Clematidis Radix and Gastrodiae Rhizoma, and the blending weight ratio of the two herbs or their herbal extracts may be 1 : 0.05 to 20, and most preferably 1 : 1.

For example, the pharmaceutical composition contains a herbal extract of Genkwae Flos, Clematidis Radix, and Gastrodiae Rhizoma, and the blending weight ratio of Genkwae Flos, Clematidis Radix, and Gastrodiae Rhizoma may be 1 : 1 to 20 : 1 to 20, preferably 1 : 5 to 15 : 5 to 15, and most preferably 1 : 10 : 10.

In addition, the pharmaceutical composition of the present invention may further include extracts of other herbs known in the art to have the same or similar effects as those exhibited by the composition of the present invention.

In addition, the pharmaceutical composition of the present invention may further include an active ingredient known in the art to have a therapeutic effect on neurodegenerative diseases. Examples include L-dopa or dopamine agonists, MAO-A and B inhibitors, neuroprotective agents, and the like.

In addition, the pharmaceutical composition of the present invention may further include an active ingredient that has an effect on diseases other than neurodegenerative diseases.

When the herbal extract of the present invention is used together with an additional active ingredient, the herbal extract and the additional active ingredient may be administered simultaneously as one formulation, or may be administered simultaneously or sequentially as separate formulations.

In addition, the pharmaceutical composition of the present invention may be used alone or in combination with methods using surgery, hormone therapy, drug therapy, or biological response regulators in order to treat neurodegenerative diseases.

The herbal extract used in the present disclosure can be obtained using a conventional extraction solvent known in the art. As the extraction solvent, a polar solvent or a non-polar solvent may be used. Polar solvents include water, a C1 to C6 alcohol (for example, methanol, ethanol, propanol, butanol, n-propanol, iso-propanol and n-butanol, etc.), acetic acid, or a mixture of the above polar solvents. Non-polar solvents include acetone, acetonitrile, ethyl acetate, methyl acetate, butyl acetate, fluoroalkane, hexane, ether, chloroform, dichloromethane, or a mixture of the above non-polar solvents.

For example, the herbal extract may be extracted with a solvent selected from the group consisting of water, a C1 to C6 alcohol, acetic acid, a mixed solvent thereof, and a non-polar solvent. Preferably, it is a 0.01% to 90% ethanol extract, and most preferably, it is a 50% to 70% ethanol extract.

The herbal extract used in the present disclosure may be extracted through hot water extraction, cold extraction, reflux cooling extraction, ultrasonic extraction, or a conventional extraction method known in the art.

As used herein, the term "extract" refers to what is commonly used in the art as a crude extract, but broadly also includes a fraction obtained by further fractionation of the extract. That is, the herbal extract includes not only those obtained using the above-described solvent, but also those obtained by additionally applying a purification process thereto. For example, the herbal extract of the present invention also includes a fraction obtained by passing the extract through an ultrafiltration membrane having a constant molecular weight cut-off value, and a fraction obtained through various additional purification methods, such as separation by various chromatographs (prepared for separation according to size, charge, hydrophobicity or affinity).

In the present disclosure, the neurodegenerative disease may be Parkinson's disease, Alzheimer's disease, Pick's disease, Huntington's disease, Lou Gehrig's disease, prion disease, Lewy body dementia, multiple system atrophy, progressive supranuclear palsy, Friedreich's ataxia, temporal lobe epilepsy, or stroke. Preferably, it is Parkinson's disease.

As used herein, the term "neurodegenerative" includes all of the transition from a normal state to a hypofunction state of a nerve cell, a genetic hypofunction, and a sporadic hypofunction, and includes the slow and continuous death of nerve cells in one part or several parts of the nervous system.

As used herein, the term "preventing" refers to any action that inhibits or delays the onset of a neurodegenerative disease by administration of the pharmaceutical composition according to the present disclosure and "treating" refers to any action that ameliorates or beneficially changes the symptoms of the subject who is suspected to have or has developed a neurodegenerative disease by administration of the pharmaceutical composition.

For example, the pharmaceutical composition may prevent or treat neurodegenerative diseases by enhancing behavioral ability.

For example, the pharmaceutical composition may prevent or treat neurodegenerative diseases by inhibiting cell death caused by toxicity in nerve cells.

For example, the pharmaceutical composition may prevent or treat neurodegenerative diseases by inhibiting the production of reactive oxygen species (ROS) in nerve cells.

For example, the pharmaceutical composition may prevent or treat neurodegenerative diseases by protecting the function of mitochondria in nerve cells.

For example, the pharmaceutical composition may prevent or treat neurodegenerative diseases by inhibiting apoptosis of nerve cells.

For example, the pharmaceutical composition may prevent or treat neurodegenerative diseases by inhibiting the production of nitric oxide (NO).

The disclosure also mentions a food composition not claimed for preventing or improving neurodegenerative diseases, the composition containing a herbal extract of two or more selected from the group consisting of Genkwae Flos, Clematidis Radix, and Gastrodiae Rhizoma.

As used herein, the term "improving" refers to any action that at least reduces a parameter, for example, the severity of a symptom, associated with a condition to be treated by administration of the composition of the present invention containing an extract.

The food composition of the present invention includes health functional foods and health foods, etc.

The food composition of the present invention can be taken for a long time.

The food composition of the present invention may include various nutrients, vitamins, minerals (electrolytes), flavoring agents such as synthetic flavoring agents and natural flavoring agents, coloring agents and enhancers (cheese, chocolate, etc.), pectic acid and salts thereof, alginic acid and salts thereof, organic acids, protective colloidal thickeners, pH adjusters, stabilizers, preservatives, glycerin, alcohols, carbonation agents used in carbonated beverages, and the like.

The disclosure mentions a feedstuff composition not claimed for preventing or improving neurodegenerative diseases, the composition containing a herbal extract of two or more selected from the group consisting of Genkwae Flos, Clematidis Radix, and Gastrodiae Rhizoma.

Hereinafter, the present invention will be described in more detail through the following examples, but the following examples are for illustrative purposes only.

### [Preparation Example 1]

### Preparation of single herbal extract

The washed and dried Genkwae Flos, Clematidis Radix, and Gastrodiae Rhizoma were used. To 75 g of each of Genkwae Flos, Clematidis Radix, and Gastrodiae Rhizoma herbs was added a 70% (v/v) aqueous ethanol solution in an amount 10 times, and extracted at room temperature for 72 hours while stirring to obtain an extract. Thereafter, each of the extracted solution was filtered and concentrated under reduced pressure at 50-65 °C, and then lyophilized to obtain a single herbal extract in a powder state.

### [Preparation Example 2]

### Preparation of mixed herbal extract of Genkwae Flos, Clematidis Radix, and Gastrodiae Rhizoma

The washed and dried Genkwae Flos, Clematidis Radix, and Gastrodiae Rhizoma herbs were mixed in a weight ratio of 1:1:1 to make a total of 75 g, and then 70% (v/v) aqueous ethanol solution in an amount 10 times was added, and extracted at room temperature for 72 hours while stirring well. Thereafter, the extracted solution was filtered and concentrated under reduced pressure at 50-65 °C, and then lyophilized to obtain a mixed herbal extract in a powder state.

### [Preparation Example 3]

### Preparation of mixed herbal extract of Clematidis Radix and Gastrodiae Rhizoma

The washed and dried Clematidis Radix and Gastrodiae Rhizoma herbs were mixed in a weight ratio of 1:1 to make a total of 80 g, and then 70% (v/v) aqueous ethanol solution in an amount 10 times was added, and extracted at room temperature for 72 hours while stirring well. Thereafter, the extracted solution was filtered and concentrated under reduced pressure at 50-65 °C, and then lyophilized to obtain a mixed herbal extract in a powder state.

### [Preparation Example 4]

### Preparation of mixed herbal extract of Genkwae Flos, Clematidis Radix, and Gastrodiae Rhizoma

Using the washed and dried Genkwae Flos, Clematidis Radix, and Gastrodiae Rhizoma, a mixed herbal extract was prepared as follows and used in the experiments of Examples 9 to 11. It was pulverized into a circular shape in the case of Genkwae Flos, or pulverized to a size that passed through a sieve of control (4,750 µm) in the case of Clematidis Radix and Gastrodiae Rhizoma, and then used in the experiment. The Genkwae Flos, Clematidis Radix, and Gastrodiae Rhizoma were mixed according to the weight ratio (w/w) described in Table 1, and then 70% (v/v) aqueous ethanol solution in an amount 10 times was added and extracted at room temperature for 72 hours. The extracted solution was filtered, and then concentrated under reduced pressure at 50-65 °C, and then lyophilized to obtain a mixed herbal extract in a powder state.

**[Table 1]**

| | **Weight ratio (w/w)** | | | **Total amount of raw herb (g)** | **Yield (%)** |
|---|---|---|---|---|---|
| | **Genkwae Flos** | **Clematidis Radix** | **Gastrodiae Rhizoma** | | |
| Preparation Example 4-1 | 1 | 1 | 1 | 1200 | 17.3 |
| Preparation Example 4-2 | 1 | 5 | 5 | 55 | 17.7 |
| Preparation Example 4-3 | 1 | 10 | 10 | 315 | 7.7 |

### [Example 1]

### Efficacy of mixed herbal extract to improve motor ability in animal model of Rotenone-induced Parkinson's disease

Rotenone is a substance used as an insecticide and inhibits mitochondrial activity by inhibiting the electron transport system of mitochondria. In this case, brain cells die and eventually motor function is reduced. Therefore, in order to verify the efficacy of the mixed herbal extract to enhance the behavioral ability, an experiment was performed to confirm the motor functionality in an animal model of Rotenone-induced Parkinson's disease.

5-week-old male C57BL/6 mice having a weight of 20~22 g (Orient Bio Inc., Republic of Korea) were used. The animals were maintained at a room temperature of 23 ± 1 °C with a 12-hour day and 12-hour night cycle according to the guidelines of NIH (NIH publication No.85-23, revised 1985). The mice were randomly divided into 7 groups as follows and an in vivo experiment was performed.
(1) Normal group (n=8)
(2) Rotenone (negative control group; Sigma-Aldrich, St. Louis, MO, USA) (n=8; intraperitoneal injection of Rotenone at a concentration of 1 mg/kg/day)
(3) Mixed herbal extract of Genkwae Flos, Clematidis Radix, and Gastrodiae Rhizoma (1:1:1 ratio) 1, 3, 10 mg/kg (n=8; oral administration of extract, intraperitoneal injection of Rotenone)
(4) Genkwae Flos extract 1, 3 mg/kg (n=8; oral administration of extract, intraperitoneal injection of Rotenone)
(5) Clematidis Radix extract 1, 3 mg/kg (n=8; oral administration of extract, intraperitoneal injection of Rotenone)
(6) Gastrodiae Rhizoma extract 1, 3 mg/kg (n=8; oral administration of extract, intraperitoneal injection of Rotenone)
(7) L-DOPA 25 mg/kg (positive control group; Sigma-Aldrich, USA) (n=8; intraperitoneal injection of L-DOPA, intraperitoneal injection of Rotenone)

After 3 days of housing, each of the 4 extracts was orally administered to mice for 21 days. In the case of L-DOPA, a positive control group, intraperitoneal (i.p.) administration was also performed for 21 days. For the groups except for the normal group, 1 mg/kg/day Rotenone dissolved in saline was intraperitoneally (i.p.) administered for 14 days from 7 day after the start of the extract administration.

In order to confirm the motor functionality, Rotarod test was performed. The Rotarod test is a test to measure running time on a rotating cylinder, and ROTA-ROD TREADMILL DJ345 (Daejong Machinery Industry Corporation, Republic of Korea) was used. Before proceeding with this experiment, the mice were trained three times to run for 5 minutes on a cylinder rotating at a speed of 5 and 15 rpm two days and one day before this experiment in order to get used to the instrument.

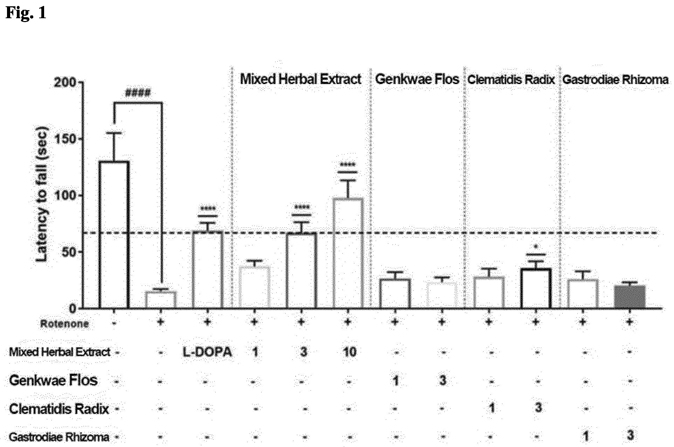

The time the mouse fell to the floor while running on a cylinder that rotates at 20 rpm was measured, and the measurement time was measured using an automatic timer attached to the apparatus, and the maximum measurement time was fixed at 300 seconds. The results are shown in Fig. 1.

In the case of the negative control group injected only with Rotenone into C57BL/6 mice, the behavioral ability was only 11.9% compared to that of the normal group to which Rotenone was not administered (88.1% decrease). When the mixed herbal extract prepared in Preparation Example 2 was administered at concentrations of 1, 3, and 10 mg/kg, the behavioral ability was enhanced to 19.0%, 44.8%, and 75.0% levels compared to that of the normal group, respectively. It was confirmed that the decrease in behavioral ability due to Rotenone treatment was inhibited. In the group treated with each single herbal extract, the behavioral ability was slightly increased or similar to that of the negative control group, so the degree of enhancement in behavioral ability was lower than that of the group administered with the mixed herbal extract. The degree of enhancement in behavioral ability shown in the positive control group administered with L-DOPA 25 mg/kg was similar to that of the group administered with the mixed herbal extract 3 mg/kg, and in the group administered with the mixed herbal extract 10 mg/kg, the degree of enhancement in behavioral ability was superior to that of the positive control group administered with L-DOPA.

The mixed herbal extract significantly enhanced the behavioral ability compared to the single herbal extract as well as the negative control group. The mixed herbal extract showed similar or excellent effects at a lower dose even when compared to the group administered with L-DOPA, a precursor of dopamine. Therefore, it was confirmed that it can be utilized to treat Parkinson's disease.

### [Example 2]

### Efficacy of mixed herbal extract to protect cytotoxicity caused by MPP+ in dopaminergic nerve cell line

In this example, the cytoprotective activity of the mixed herbal extract was evaluated. In order to confirm whether the mixed herbal extract of Genkwae Flos, Clematidis Radix, and Gastrodiae Rhizoma have the effect of protecting nerve cell damage due to MPP+ treatment, WST-1 assay was performed using the SH-SY5Y cell line.

Specifically, human neuroblastoma SH-SY5Y cells were cultured under RPMI 1640 medium (Gibco^{™}, USA) medium containing 10% fetal bovine serum (FBS, Gibco^{™}, USA) and antibiotics (1% Penicillin, Streptomycin [P/S], Gibco-BRL, USA). The incubator was maintained at a temperature of 37 °C, and a gas mixture of 95% air and 5% CO2 was continuously supplied to provide appropriate conditions for cell culture. The 96-well plate was coated with PDL (Poly-D-lysine, Sigma-Aldrich, USA) at 1X concentration condition, and then washed with PBS and dried, and then the cells were cultured. The cells were cultured 24 hours before the experiment to 5x10⁴ cells/well. When an appropriate number of cells were cultured, the medium was replaced with a medium containing 0.5% FBS, and then further cultured for 24 hours to differentiate the cells.

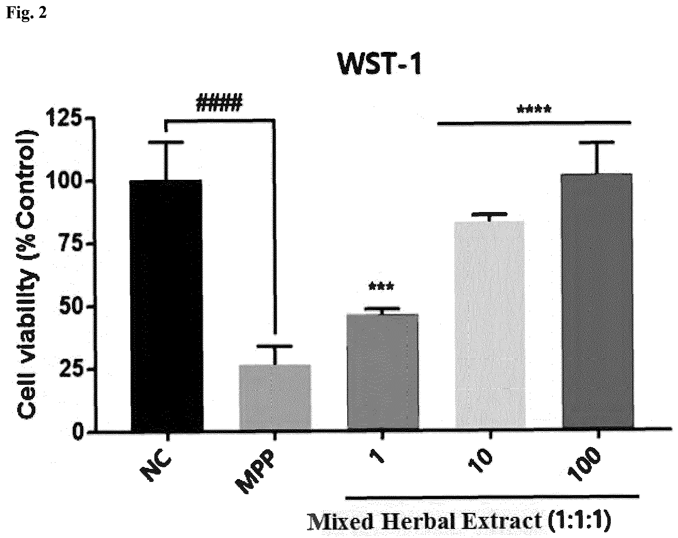

In a 96-well plate in which the SH-SY5Y cell line was cultured, the cells were treated with the mixed herbal extract of Genkwae Flos, Clematidis Radix, and Gastrodiae Rhizoma prepared in Preparation Example 2 at concentrations of 1, 10, and 100 µg/ml for 30 minutes, and then treated with 2 mM MPP+. After 48 hours of MPP+ treatment, 100 µl of 10% WST-1 solution was added to each well of a 96-well plate. After reacting for 30 minutes in a 37 °C incubator, the absorbance was measured at wavelength values of 450 and 630 nm. The results are shown in Fig. 2.

As a result of treatment with MPP+, it was confirmed that the cell viability was 26.4% in the negative control group compared to the MPP+ untreated group (normal group; NC), and cell death was highly induced. When the cells were treated with the mixed herbal extract at concentrations of 1, 10, and 100 µg/ml, cell death due to MPP+ was inhibited in a concentration-dependent manner at all concentrations (cell viability levels of 46.3%, 83.1%, and 101.8% compared to that of the normal group were confirmed). In particular, in the case of the 100 µg/ml concentration treatment group, cell death was inhibited to the level of the MPP+ untreated group (normal group; NC) (i.e., 100% cell viability). Therefore, it was confirmed that the cytoprotective activity of the mixed herbal extract was very excellent.

### [Example 3]

### Efficacy of mixed herbal extract to reduce production of reactive oxygen species caused by MPP+ in dopaminergic nerve cell line

In this example, when treated with the SH-SY5Y neurotoxin substance MPP+, the ability of the mixed herbal extract of Genkwae Flos, Clematidis Radix, and Gastrodiae Rhizoma to reduce reactive oxygen species (ROS) was confirmed. Using DCF-DA (2',7'-dichlorodihydrofluorescein diacetate, Sigma-Aldrich, USA) that is converted to DCF in cells by reactive oxygen species and emits green fluorescence, the levels of reactive oxygen species in the SH-SY5Y cells cultured in Example 2 above were measured.

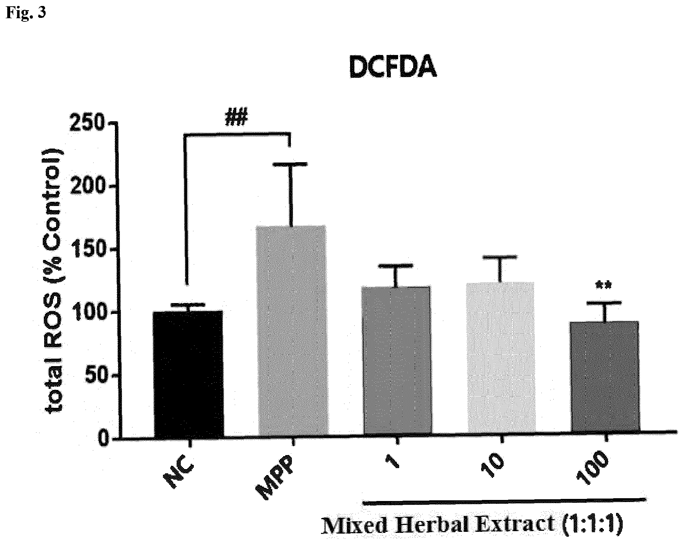

Specifically, the SH-SY5Y cells were treated with the mixed herbal extract of Genkwae Flos, Clematidis Radix, and Gastrodiae Rhizoma prepared in Preparation Example 2 at concentrations of 1, 10, and 100 µg/ml for 30 minutes and then treated with 2 mM MPP+. After 48 hours of MPP+ treatment, the cells were treated with DCF-DA at a concentration of 10 µM for 30 minutes in a 96-well plate. After washing once with PBS, fluorescence was measured in the wavelength region of 485 and 530 nm. For wavelength correction, Hoechst 33342 (ThermoFisher, USA) was dissolved in tertiary distilled water at 10 mg/ml, and then the cells were treated therewith at a ratio of 1:2000 to measure fluorescence at wavelengths of 350 and 461 nm. The results are shown in Fig. 3.

As a result of treatment with MPP+, reactive oxygen species was increased in the negative control group (MPP) treated only with MPP+ compared to the MPP+ untreated group (normal group; NC). When the cells were treated with the mixed herbal extract for each concentration, the production of reactive oxygen species was reduced compared to the negative control group (MPP). In particular, in the case of the 100 µg/ml concentration treatment group, reactive oxygen species was inhibited to the level of the MPP+ untreated group (normal group; NC). Therefore, it was confirmed that the reactive oxygen species inhibitory ability of the mixed herbal extract was excellent.

### [Example 4]

### Efficacy of mixed herbal extract to improve mitochondrial functional impairment caused by MPP+ in dopaminergic nerve cell line

In this example, when treated with the SH-SY5Y neurotoxin substance MPP+, the effect of the mixed herbal extract of Genkwae Flos, Clematidis Radix, and Gastrodiae Rhizoma to protect the mitochondrial function was analyzed. The function of mitochondria was analyzed by measuring the fluorescence level using TMRE (Tetramethylrhodamine, Ethyl Ester, Perchlorate, Sigma-Aldrich, 87917) that is absorbed into normal mitochondrial cells and emits fluorescence.

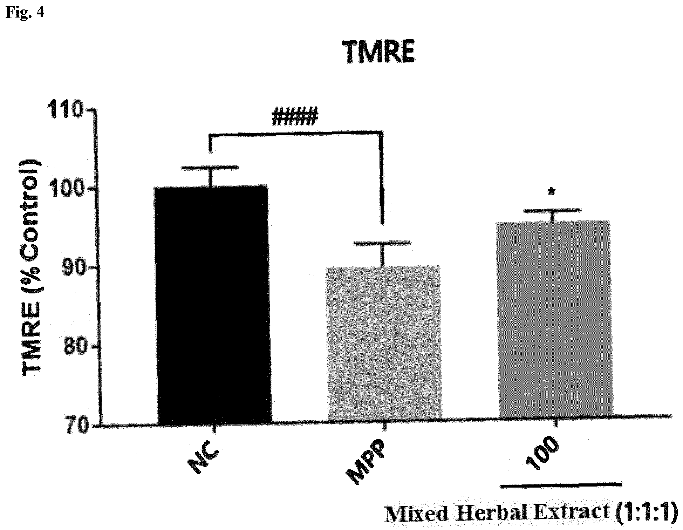

The SH-SY5Y cells cultured in Example 2 above were treated with the mixed herbal extract of Genkwae Flos, Clematidis Radix, and Gastrodiae Rhizoma prepared in Preparation Example 2 at a concentration of 100 µg/ml for 30 minutes and then treated with 2 mM MPP+ for 48 hours. Thereafter, the cells were treated with TMRE at a concentration of 0.5 µM for 30 minutes. After washing once with PBS, 100 µl of PBS was added to each well. Fluorescence was measured in the wavelength region of 549 and 575 nm, and the results are shown in Fig. 4.

As a result of treatment with MPP+, the loss of mitochondrial function was confirmed in the negative control group (MPP) treated only with MPP+ compared to the MPP+ untreated group (normal group; NC). When the cells were treated with the mixed herbal extract at a concentration of 100 µg/ml, the loss of mitochondrial function due to MPP+ treatment was inhibited. In particular, the mixed herbal extract inhibited the loss of mitochondrial function to the level of the MPP+ untreated group (normal group; NC), confirming that the mitochondrial function recovery ability of the extract was excellent. Therefore, it was confirmed that the mitochondrial function recovery ability of the mixed herbal extract was excellent.

### [Example 5]

### Efficacy of mixed herbal extract to reduce cell death caused by MPP+ in dopaminergic nerve cell line

In this example, when treated with the SH-SY5Y neurotoxin substance MPP+, the effect of the mixed herbal extract of Genkwae Flos, Clematidis Radix, and Gastrodiae Rhizoma to reduce apoptosis in cells was analyzed.

Caspase, a type of cysteine protease, is an essential protein for programmed apoptosis called cell suicide. Caspase-activated apoptosis induces cell suicide, leading to the death of the related cells, and brain cell apoptosis in Parkinson's disease exacerbates the disease. When caspase reacts with Caspase-Glo reagent containing DEVD luciferin, it reacts with ATP to emit light.

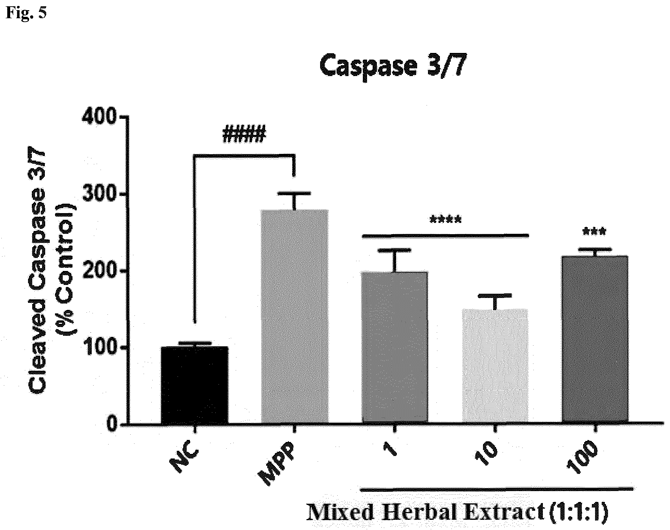

The SH-SY5Y cells cultured in Example 2 above were treated with the mixed herbal extract of Genkwae Flos, Clematidis Radix, and Gastrodiae Rhizoma prepared in Preparation Example 2 at concentrations of 1, 10, and 100 µg/ml for 30 minutes and treated with 2 mM MPP+ for 48 hours. Thereafter, Caspase-Glo buffer and substrate (Promega, USA) were mixed, 50 µl was added to each well, and the cells were treated in a condition without light for 30 minutes at room temperature. Thereafter, the values were measured in the wavelength region of 490 and 570 nm. For wavelength correction, Hoechst 33342 (ThermoFisher, USA) was dissolved in tertiary distilled water at 10 mg/ml and then added at a ratio of 1:2000 to measure at wavelengths of 350 and 461 nm. The results are shown in Fig. 5.

As a result of treatment with MPP+, 279% of caspase was measured in the negative control group (MPP) treated only with MPP+ compared to the MPP+ untreated group (normal group; NC), and apoptosis was induced at a high level. When the cells were treated with the mixed herbal extract at concentrations of 1, 10, and 100 µg/ml, the increase in caspase compared to that of the normal group was reduced by 29.0%, 46.5%, and 21.9% compared to that of the negative control group, indicating that apoptosis caused by MPP+ treatment was inhibited. Through the above results, it was confirmed that the apoptosis inhibitory ability of the extract was excellent.

### [Example 6]

### Efficacy of mixed herbal extract to inhibit inflammatory response caused by LPS in microglia cell line

In order to confirm the anti-inflammatory efficacy of the mixed herbal extract of Genkwae Flos, Clematidis Radix, and Gastrodiae Rhizoma, the change in nitric oxide (NO) content in BV2 cells, mouse microglia cells, was measured using the Griess assay.

BV2 cells were cultured under DMEM medium (Gibco^{™}, USA) medium containing fetal bovine serum (FBS, Gibco^{™}, USA) and antibiotics (1% Penicillin, Streptomycin [P/S] Gibco-BRL, USA). Specifically, the incubator was maintained at a temperature of 37 °C, and a gas mixture of 95% air and 5% CO2 was continuously supplied to provide appropriate conditions for cell culture. The cells were cultured in a 12-well plate 24 hours before the experiment to 3x10⁴ cells/well.

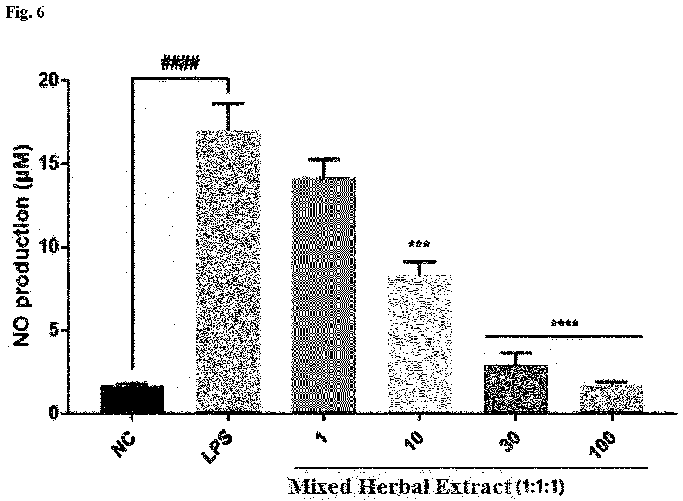

The prepared 12-well plate was pretreated with the mixed herbal extract of Genkwae Flos, Clematidis Radix, and Gastrodiae Rhizoma prepared in Preparation Example 2 at concentrations of 1, 10, 30, and 100 µg/ml for 1 hour, and then treated with LPS at a concentration of 1 µg/ml for 24 hours. Thereafter, the supernatant was taken and the change in the nitric oxide content was evaluated by measuring the absorbance at wavelength values of 520 and 550 nm through the Griess assay kit (G2930, Promega, USA). The results are shown in Fig. 6.

As a result of LPS treatment in BV2 microglia cells, nitric oxide was produced at a high level (1123%) in the negative control group (LPS) treated only with LPS compared to the LPS untreated group (normal group; NC). When the cells were treated with the mixed herbal extract at concentrations of 1, 10, 30, and 100 µg/ml, the increase in nitric oxide production was reduced at all concentrations. In particular, when the cells were treated with the mixed herbal extract at concentrations of 10, 30, and 100 µg/ml, the increase in nitric oxide production was reduced by 56.4%, 91.4%, and 99.5% compared to that of the negative control group. Therefore, it was confirmed that the LPS-induced inflammatory response inhibitory ability of the mixed herbal extract was excellent.

### [Example 7]

### Efficacy of mixed herbal extract to improve motor ability in animal model of Rotenone-induced Parkinson's disease

5-week-old male C57BL/6 mice having a weight of 20~22 g (Orient Bio Inc., Republic of Korea) were used. The animals were maintained at a room temperature of 23 ± 1 °C under a condition of a 12-hour day and 12-hour night cycle according to the guidelines of NIH (NIH publication No.85-23, revised 1985). The mice were randomly divided into 5 groups as follows and an in vivo experiment was performed.
(1) Normal group (n=8)
(2) Rotenone (negative control group; Sigma-Aldrich, St. Louis, MO, USA) (n=8; intraperitoneal injection of Rotenone at a concentration of 1 mg/kg/day)
(3) Mixed herbal extract of Genkwae Flos, Clematidis Radix, and Gastrodiae Rhizoma (1:1:1 ratio) 5 mg/kg (n=8; oral administration of extract, intraperitoneal injection of Rotenone)
(4) Mixed herbal extract of Clematidis Radix and Gastrodiae Rhizoma (1:1 ratio) 5 mg/kg (n=8; oral administration of extract, intraperitoneal injection of Rotenone)
(5) L-DOPA 25 mg/kg (positive control group; Sigma-Aldrich, USA) (n=8; intraperitoneal injection of L-DOPA, intraperitoneal injection of Rotenone)

After 3 days of housing, each of the mixed herbal extract of two types of herbs was orally administered to mice for 21 days. In the case of L-DOPA, a positive control group, intraperitoneal (i.p.) administration was also performed for 21 days. For the groups except for the normal group, 1 mg/kg/day Rotenone dissolved in saline was intraperitoneally (i.p.) administered for 14 days from 7 day after the start of the extract administration.

In order to confirm the motor functionality, Rotarod test was performed. The Rotarod test is a test to measure running time on a rotating cylinder, and ROTA-ROD TREADMILL DJ345 (Daejong Machinery Industry Corporation, Republic of Korea) was used. Before proceeding with this experiment, the mice were trained three times to run for 5 minutes on a cylinder rotating at a speed of 5 and 15 rpm two days and one day before this experiment in order to get used to the instrument.

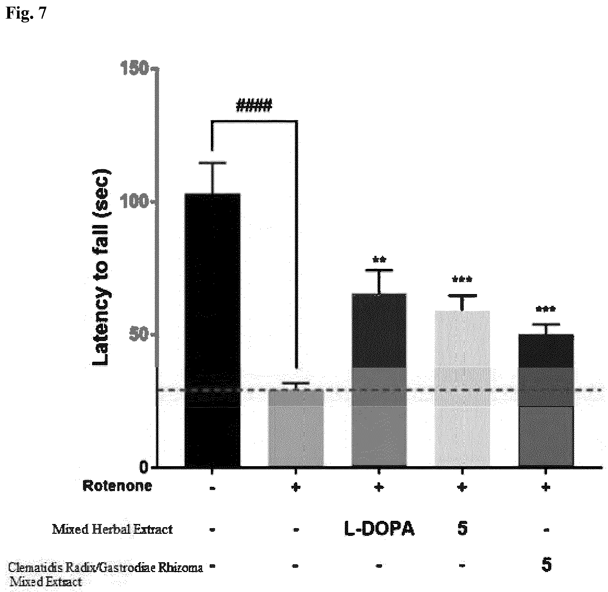

The time the mouse fell to the floor while running on a cylinder that rotates at 20 rpm was measured, and the measurement time was measured using an automatic timer attached to the apparatus, and the maximum measurement time was fixed at 300 seconds. The results are shown in Fig. 7.

In the case of the negative control group injected only with Rotenone into C57BL/6 mice, the behavioral ability was only 28.6% compared to that of the normal group to which Rotenone was not administered (71.4% decrease). When the mixed herbal extract of Genkwae Flos, Clematidis Radix, and Gastrodiae Rhizoma prepared in Preparation Example 2 (indicated as "mixed herbal extract" in Fig. 7) and the mixed herbal extract of Clematidis Radix and Gastrodiae Rhizoma prepared in Preparation Example 3 (indicated as "Clematidis Radix/Gastrodiae Rhizoma mixed extract" in Fig. 7) were administered at a concentration of 5 mg/kg, respectively, the behavioral ability was enhanced to 57.5% and 48.4% levels compared to that of the normal group, respectively. It was confirmed that the decrease in behavioral ability due to Rotenone treatment was inhibited.

### [Example 8]

### Efficacy of mixed herbal extract to inhibit inflammatory response caused by LPS in microglia cell line

In order to confirm the anti-inflammatory efficacy of the mixed herbal extract of Genkwae Flos, Clematidis Radix, and Gastrodiae Rhizoma and the mixed herbal extract of Clematidis Radix and Gastrodiae Rhizoma, the change in nitric oxide (NO) content in BV2 cells, mouse microglia cells, was measured using the Griess assay.

BV2 cells were cultured under DMEM medium (Gibco^{™}, USA) medium containing fetal bovine serum (FBS, Gibco^{™}, USA) and antibiotics (1% Penicillin, Streptomycin [P/S] Gibco-BRL, USA). Specifically, the incubator was maintained at a temperature of 37 °C, and a gas mixture of 95% air and 5% CO2 was continuously supplied to provide appropriate conditions for cell culture. The cells were cultured in a 12-well plate 24 hours before the experiment to 3x10⁴ cells/well.

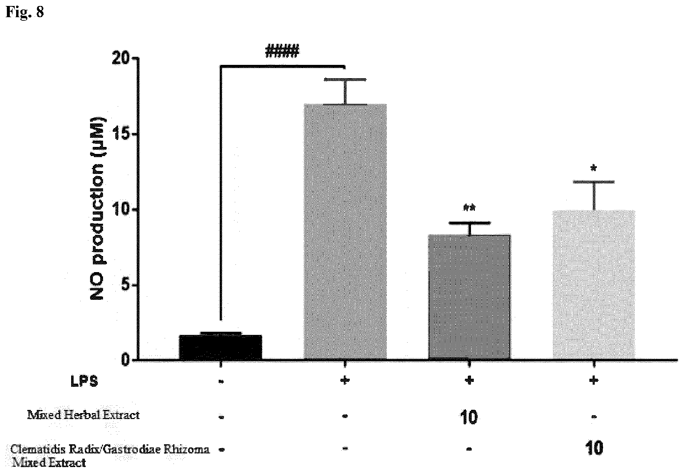

The prepared 12-well plate was pretreated with the mixed herbal extract of Genkwae Flos, Clematidis Radix, and Gastrodiae Rhizoma prepared in Preparation Example 2 and the mixed herbal extract of Clematidis Radix and Gastrodiae Rhizoma prepared in Preparation Example 3 at a concentration of 10 µg/ml for 1 hour, and then treated with LPS at a concentration of 1 µg/ml for 24 hours. Thereafter, the supernatant was taken and the change in the nitric oxide content was evaluated by measuring the absorbance at wavelength values of 520 and 550 nm through the Griess assay kit (G2930, Promega, USA). The results are shown in Fig. 8.

As a result of LPS treatment in BV2 microglia cells, nitric oxide was produced at a high level (1123%) in the negative control group (LPS) treated only with LPS compared to the LPS untreated group (normal group; NC). When the cells were treated with the mixed herbal extract of Genkwae Flos, Clematidis Radix, and Gastrodiae Rhizoma (indicated as "mixed herbal extract" in Fig. 8) and the mixed herbal extract of Clematidis Radix and Gastrodiae Rhizoma (indicated as "Clematidis Radix/Gastrodiae Rhizoma mixed extract" in Fig. 8) at a concentration of 10 µg/ml, respectively, the increase in nitric oxide production was reduced, and in particular, it was reduced by 56.4% and 54.1% compared to that of the negative control group, respectively. Therefore, it was confirmed that the LPS-induced inflammatory response inhibitory ability of the mixed herbal extract and Clematidis Radix/Gastrodiae Rhizoma extract was excellent.

### [Example 9]

### Comparison of vomiting occurrence of mixed herbal extract by blending ratio in Beagle dog

In this example, changes in general symptom such as body weight change and vomiting in Beagle dog after oral administration of the mixed herbal extract of Genkwae Flos, Clematidis Radix, and Gastrodiae Rhizoma at a blending ratio of 1:1:1, 1:5:5 or 1:10:10 were observed.

Beagle dogs used in the test were 5 male dogs and 5 female dogs (male G1101, G1201, G1301, G1401, G1501; female G2101, G2201, G2301, G2401, G2501) of 9-14-month-old (Beijing Marshall Biotechnology Co., Ltd., China), and the test substances were prepared on the day of administration by weighing the lyophilized powder prepared in Preparation Examples 4-1 (a blending ratio of 1:1:1), 4-2 (1:5:5) and 4-3 (1:10:10) according to the required amount and suspending in water for injection (JW Pharmaceutical, Republic of Korea), or used by filling in general gelatin capsules or enteric capsules the day before administration. The amount of the administered solution was 5 ml/kg body weight. The test design was carried out as shown in Table 2, and each test was performed with a wash-out period of at least 5 days or longer.

**[Table 2]**

| **Test No.** | **Subject No.** | | **Administered substance** | **Administration period and dose** | **Remarks** |
|---|---|---|---|---|---|
| | **Male** | **Female** | | | |
| 1 | G1101 | G2101 | Preparation Example 4-1 | 50 mg/kg single dose | |
| 2 | G1101 | G2101 | Preparation Example 4-1 | 50 mg/kg single dose | filled into general gelatin capsules and administered |
| 3 | G1201 | G2201 | Preparation Example 4-1 | 50 mg/kg single dose | filled into enteric capsules and administered |
| 4 | G1101, G1201 | G2101, G2201 | Preparation Example 4-1 | 25 mg/kg once a day for 4 days | |
| 5 | G1101 | G2101 | Preparation Example 4-2 | 41.25 mg/kg once a day for 5 days | |
| 6 | G1301, G1401 | G2301, G2401 | Preparation Example 4-3 | 50 mg/kg single dose | |
| 7 | G1501 | G2501 | Preparation Example 4-3 | 100 mg/kg once a day for 7 days | |

The results of measuring the body weight of the animals for each test (single dose administration: the next day after administration, repeated administration: the next day after the last administration) are shown in Table 3 below. As shown in Table 3 below, no change in body weight was observed by administration of the test substance in both male and female dogs.

**[Table 3]**

| **Body weight (kg)** | **Subject No** | **Test No.** | | | | | | |
|---|---|---|---|---|---|---|---|---|
| | | **1** | **2** | **3** | **4** | **5** | **6** | **7** |
| Male | G1101 | 7.19 | 7.51 | - | 7.94 | 8.75 | - | |
| Female | G2101 | 6.49 | 6.51 | - | 6.93 | 7.2 | - | |
| Male | G1201 | - | - | 8.46 | 8.98 | - | - | |
| Female | G2201 | - | - | 5.88 | 6.12 | - | - | |
| Male | G1301 | - | - | - | - | - | 9.79 | |
| Female | G2301 | - | - | - | - | - | 7.23 | |
| Male | G1401 | - | - | - | - | - | 7.73 | |
| Female | G2401 | - | - | - | - | - | 6.68 | |
| Male | G1501 | - | - | - | - | - | - | 8.96 |
| Female | G2501 | - | - | - | - | - | - | 7.06 |

| | | | | | | | | |
|---|---|---|---|---|---|---|---|---|
| (- : No measurement result) | | | | | | | | |

In addition, the results of observing the general symptoms (vomiting, fecal status, etc.) of the animals for each test are shown in Table 4 below.

**[Table 4]**

| **Test No.** | **Administered substance** | **Administration period and dose** | **Result of observing general symptoms** |
|---|---|---|---|
| 1 | Preparation Example 4-1 | 50 mg/kg single dose | Female: observation of vomiting for 30 minutes to 1 hour after administration |
| | | | Male: observation of vomiting for 1 hour after administration |
| 2 | Preparation Example 4-1 | 50 mg/kg single dose (filled into general gelatin capsule) | Female: observation of vomiting for 1 hour after administration |
| | | | Male: observation of vomiting for 30 minutes after administration |
| 3 | Preparation Example 4-1 | 50 mg/kg single dose (filled into enteric capsule) | Both female and male: observation of vomiting for 30 minutes to 4 hours after administration |
| 4 | Preparation Example 4-1 | 25 mg/kg once a day for 4 days | Female: observation of vomiting for 2-3 days after administration |
| | | | Male: observation of vomiting for 2-4 days after administration |
| 5 | Preparation Example 4-2 | 41.25 mg/kg once a day for 5 days | Both female and male: observation of vomiting on the first day of administration, no vomiting thereafter |
| 6 | Preparation Example 4-3 | 50 mg/kg single dose | Both female and male: no vomiting |
| 7 | Preparation Example 4-3 | 100 mg/kg once a day for 7 days | Female only: observation of vomiting on the first day of administration, no vomiting thereafter |

As shown in Table 4 above, in all tests (Test Nos. 1 to 4) in which Preparation Example 4-1 containing Genkwae Flos, Clematidis Radix, and Gastrodiae Rhizoma in a 1:1:1 blending ratio was administered once or repeatedly, it was confirmed that vomiting occurred in both male and female dogs. In addition, in the test (Test No. 5) in which Preparation Example 4-2 containing Genkwae Flos, Clematidis Radix, and Gastrodiae Rhizoma in a 1:5:5 blending ratio was administered repeatedly, it was confirmed that vomiting occurred on the first day in both male and female dogs.

On the other hand, in the case of the test (Test No. 6) in which Preparation Example 4-3 containing Genkwae Flos, Clematidis Radix, and Gastrodiae Rhizoma in a 1:10:10 blending ratio was administered as a single dose, it was confirmed that abnormal symptoms including vomiting were not observed in both male and female dogs unlike the test (Test Nos. 1 to 3) in which Preparation Example 4-1 was administered at the same dose.

In addition, in the case of the test (Test No. 7) in which the mixed herbal extract of Preparation Example 4-3 (a blending ratio of 1:10:10) was administered repeatedly, it was confirmed that it exhibited a relatively mild degree of vomiting occurrence despite the conditions of the higher administration dose and administration period compared to the test (Test Nos. 4 and 5) in which the mixed herbal extract of Preparation Examples 4-1 (a blending ratio of 1:1:1) and 4-2 (a blending ratio of 1:5:5) was administered repeatedly.

Therefore, it was confirmed that side effects such as vomiting induction were significantly improved in the group administered with the mixed herbal extract of Genkwae Flos, Clematidis Radix, and Gastrodiae Rhizoma in a blending ratio of 1:10:10.

### [Example 10]

### Comparison of efficacy of mixed herbal extract to protect cytotoxicity caused by MPP+ or 6-hydroxydopamine by blending ratio

In order to confirm whether the mixed herbal extract of Genkwae Flos, Clematidis Radix, and Gastrodiae Rhizoma by blending ratio has an effect of protecting nerve cell damage caused by MPP+ or 6-hydroxydopamine (6-OHDA), MTT assay was performed using the SH-SY5Y cell line.

Specifically, human neuroblastoma SH-SY5Y cells were cultured under DMEM medium (Gibco^{™}, USA) medium containing 10% fetal bovine serum (FBS, Gibco^{™}, USA) and antibiotics (1% Penicillin, Streptomycin [P/S], Gibco-BRL, USA). The incubator was maintained at a temperature of 37 °C, and a gas mixture of 95% air and 5% CO2 was continuously supplied to provide appropriate conditions for cell culture. The cells were cultured 24 hours before the experiment to 4x10⁵ cells/ml. When an appropriate number of cells were cultured, the medium was replaced with a medium containing 0.5% FBS, and then further cultured for 20 hours to differentiate the cells.

In a 96-well plate in which the SH-SY5Y cell line was cultured, the cells were treated for 4 hours with the 1:1:1, 1:5:5, and 1:10:10 mixed herbal extracts of Genkwae Flos, Clematidis Radix, and Gastrodiae Rhizoma prepared in Preparation Examples 4-1, 4-2 and 4-3 at concentrations of 3, 10, and 30 µg/ml, respectively, and then treated with 1 mM MPP+. After 44 hours of MPP+ treatment, 100 µl of MTT solution at a concentration of 1 mg/ml was added to each well of a 96-well plate. It was reacted for 1 hour in a 37 °C incubator, and MTT solution was removed, and then 100 µl of 100% DMSO was added to each well and shaken for about 15 minutes, and then the absorbance was measured at a wavelength value of 570 nm.

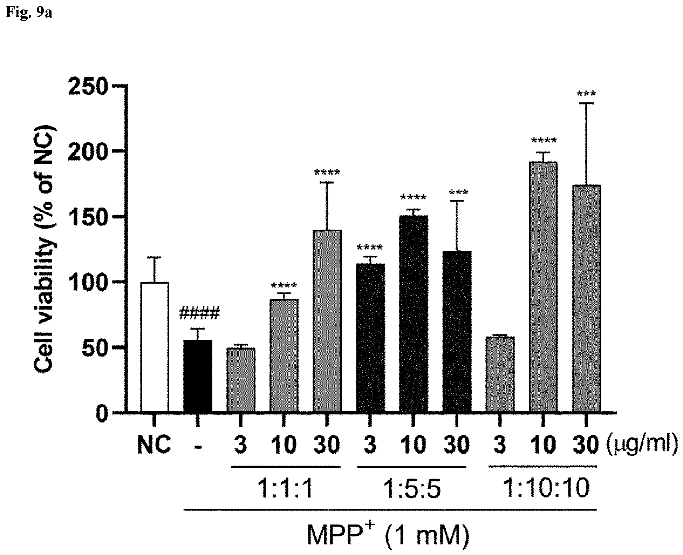

As a result, as shown in Fig. 9a, in the negative control group treated with MPP+, the cell viability was 55.6% compared to the MPP+ untreated group (normal group; NC). Therefore, statistically significant cell death was confirmed. In addition, when the cells were treated with the mixed herbal extracts having the blending ratios of 1:1:1, 1:5:5, and 1:10:10 at concentrations of 3, 10, and 30 µg/ml, respectively, in the 1:1:1 mixed herbal extract (10, 30 µg/ml), the 1:5:5 mixed herbal extract (3, 10, and 30 µg/ml), and the 1:10:10 mixed herbal extract (10, 30 µg/ml), cell death due to MPP+ was inhibited in a concentration-dependent manner. In particular, the group treated with the 1:10:10 mixed herbal extract at concentrations of 10 and 30 µg/ml showed the highest cytoprotective activity at the same concentration compared to the group treated with the 1:1:1 or 1:5:5 mixed herbal extract. Therefore, it was confirmed that it has an excellent efficacy of protecting nerve cells.

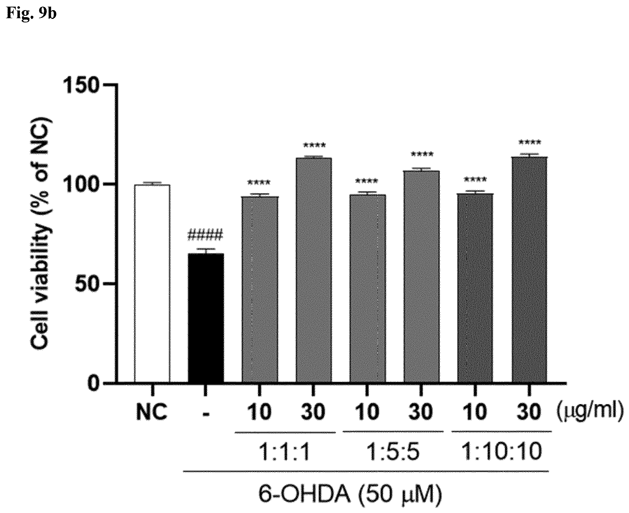

In addition, the same cells were treated for 1 hour with the 1:1:1, 1:5:5, and 1:10:10 mixed herbal extracts of Genkwae Flos, Clematidis Radix, and Gastrodiae Rhizoma prepared in Preparation Examples 4-1, 4-2, and 4-3 at concentrations of 10 and 30 µg/ml, respectively, and then treated with 50 µM 6-OHDA instead of MPP+ as a nerve cell toxicity inducing substance. After 23 hours of 6-OHDA treatment, the absorbance was measured in the same manner as MPP+, and the results are shown in Fig. 9b. The cytoprotective effect on 6-OHDA neurotoxicity was similar in the mixed herbal extract according to the blending ratios of 1:1:1, 1:5:5, and 1:10:10.

### [Example 11]

### Efficacy of mixed herbal extract to improve motor ability in animal model of MPTP-induced Parkinson's disease

MPTP (1-methyl-4-phenyl-1,2,3,6-tetrahydropyridine) inhibits mitochondrial activity by inhibiting the electron transport system of mitochondria. In this case, brain cells die and eventually motor function is reduced. Therefore, in order to verify the efficacy of the mixed herbal extract to enhance the behavioral ability, an experiment was performed to confirm the motor functionality in an animal model of MPTP-induced Parkinson's disease.

8-week-old male C57BL/6J mice (DBL Inc., Republic of Korea) were used in the experiment after an acclimatization period of 5 days. The animals were maintained at a room temperature of 23 ± 1 °C with a 12-hour day and 12-hour night cycle according to the guidelines of NIH (NIH publication No.85-23, revised 1985). The mice were randomly divided into 4 groups as follows and an in vivo experiment was performed.
(1) Normal group (n=8)
(2) MPTP (negative control group; Sigma-Aldrich, St. Louis, MO, USA) (n=8; intraperitoneal administration at a concentration of 30 mg/kg/day once a day for 5 days)
(3) MPTP + 1:5:5 mixed herbal extract of Genkwae Flos, Clematidis Radix, and Gastrodiae Rhizoma (Preparation Example 4-2) 30 mg/kg (n=8; oral administration of extract once a day for 12 days, intraperitoneal administration of MPTP)
(4) MPTP + 1:10:10 mixed herbal extract of Genkwae Flos, Clematidis Radix, and Gastrodiae Rhizoma (Preparation Example 4-3) 30 mg/kg (n=8; oral administration of extract once a day for 12 days, intraperitoneal administration of MPTP)

After the acclimatization period, each of the extracts was orally administered to mice for 12 days. In addition, for the groups except for the normal group, 30 mg/kg/day MPTP dissolved in saline was intraperitoneally administered (intraperitoneal injection, i.p.) for 5 days from the start of the extract administration.

Pole test was performed to confirm the functionality for rotational motion. Pole test is a test to measure the time it takes for the mouse to rotate in the downward direction by placing the mouse on the upper end of the vertical bar. Before proceeding with this experiment, the mice were trained once in the same manner one day before this experiment in order to get used to the instrument.

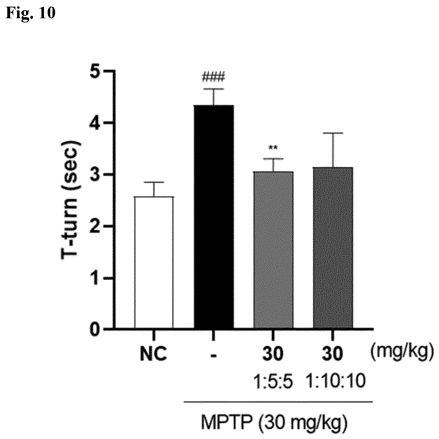

The mouse was placed on the upper end of the vertical bar, and the time it took for the mouse to rotate its body in the downward direction was measured, and the results are shown in Fig. 10.

As can be seen in Fig. 10, in the case of the negative control group injected only with MPTP into C57BL/6 mice, more time was required by 68.2% compared to that of the normal group in the T-turn required time measurement result of the Pole test. On the other hand, when the 1:5:5 or 1:10:10 mixed herbal extract of Genkwae Flos, Clematidis Radix, and Gastrodiae Rhizoma was administered at a concentration of 30 mg/kg, respectively, the required time was reduced by 49.7% and 46.4%, respectively, compared to that of the negative control group. Therefore, it was confirmed that the decrease in rotational motion ability caused by MPTP treatment was inhibited.

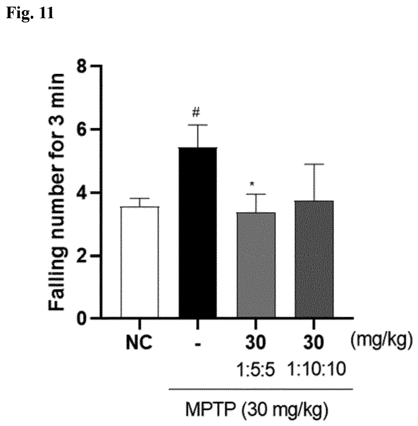

In addition, the Rotarod test was performed to evaluate the overall motor ability. The Rotarod test is a test that evaluates motor ability by placing an experimental animal on a rotating cylinder. The Rotarod test was performed using the ROTA-ROD TREADMILL (Jeung Do Bio & Plant Co., Ltd., Republic of Korea). Before proceeding with this experiment, the mice were trained twice to run for 3 minutes on a cylinder rotating at a speed of 9 rpm one day before the experiment in order to get used to the instrument. In this experiment, the number of times the mouse fell to the floor after running for 3 minutes on a cylinder rotating at 11 rpm was measured, and the results are shown in Fig. 11.

As can be seen in Fig. 11, as a result of measuring the number of times the mouse fell from the cylinder for 3 minutes, it was increased by 52.6% in the case of the negative control group injected only with MPTP compared to that of the normal group. In contrast, in the group administered with 30 mg/kg of the 1:5:5 or 1:10:10 mixed herbal extract of Genkwae Flos, Clematidis Radix, and Gastrodiae Rhizoma, respectively, the number of times the mouse fell from the cylinder was reduced by 57.9% and 47.4%, respectively, compared to that of the negative control group. Therefore, it was confirmed that the decrease in overall motor ability due to MPTP treatment was inhibited.

Eventually, through the Parkinson's disease animal model test, it was confirmed that the mixed herbal extract of Genkwae Flos, Clematidis Radix, and Gastrodiae Rhizoma of the present invention had excellent efficacy of improving motor ability.

As can be seen in Fig. 10, in the case of the negative control group injected only with MPTP into C57BL/6 mice, more time was required by 68.2% compared to that of the normal group in the T-turn required time measurement result of the Pole test. On the other hand, when the 1:5:5 or 1:10:10 mixed herbal extract of Genkwae Flos, Clematidis Radix, and Gastrodiae Rhizoma was administered at a concentration of 30 mg/kg, respectively, the required time was reduced by 49.7% and 46.4%, respectively, compared to that of the negative control group. Therefore, it was confirmed that the decrease in rotational motion ability caused by MPTP treatment was inhibited.

In addition, the Rotarod test was performed to evaluate the overall motor ability. The Rotarod test is a test that evaluates motor ability by placing an experimental animal on a rotating cylinder. The Rotarod test was performed using the ROTA-ROD TREADMILL (Jeung Do Bio & Plant Co., Ltd., Republic of Korea). Before proceeding with this experiment, the mice were trained twice to run for 3 minutes on a cylinder rotating at a speed of 9 rpm one day before the experiment in order to get used to the instrument. In this experiment, the number of times the mouse fell to the floor after running for 3 minutes on a cylinder rotating at 11 rpm was measured, and the results are shown in Fig. 11.

As can be seen in Fig. 11, as a result of measuring the number of times the mouse fell from the cylinder for 3 minutes, it was increased by 52.6% in the case of the negative control group injected only with MPTP compared to that of the normal group. In contrast, in the group administered with 30 mg/kg of the 1:5:5 or 1:10:10 mixed herbal extract of Genkwae Flos, Clematidis Radix, and Gastrodiae Rhizoma, respectively, the number of times the mouse fell from the cylinder was reduced by 57.9% and 47.4%, respectively, compared to that of the negative control group. Therefore, it was confirmed that the decrease in overall motor ability due to MPTP treatment was inhibited.

Eventually, through the Parkinson's disease animal model test, it was confirmed that the mixed herbal extract of Genkwae Flos, Clematidis Radix, and Gastrodiae Rhizoma of the present invention had excellent efficacy of improving motor ability.

## Claims

1. A pharmaceutical composition for use in preventing or treating neurodegenerative diseases, the composition containing herbal extracts of Genkwae Flos, Clematidis Radix, and Gastrodiae Rhizoma,
wherein the herbal extracts are 70% ethanol extracts,
wherein the blending weight ratio of Genkwae Flos, Clematidis Radix, and Gastrodiae Rhizoma is 1 : 10 : 10, and
wherein the neurodegenerative disease is Parkinson's disease.

2. The pharmaceutical composition for use in preventing or treating neurodegenerative diseases according to claim 1, **characterized in that** the herbal extract includes extracts from each of Genkwae Flos, Clematidis Radix, and Gastrodiae Rhizoma, or is extracted from a mixture of Genkwae Flos, Clematidis Radix, and Gastrodiae Rhizoma.

3. The pharmaceutical composition for use in preventing or treating neurodegenerative diseases according to claim 1, **characterized in that** the pharmaceutical composition:
(a) enhances behavioral ability
(b) inhibits cell death caused by toxicity in nerve cells
(c) inhibits the production of reactive oxygen species (ROS) in nerve cells
(d) protects the function of mitochondria in nerve cells
(e) inhibits apoptosis of nerve cells; or
(f) inhibits the production of nitric oxide (NO).

## Patentansprüche

1. Pharmazeutische Zusammensetzung zur Verwendung bei der Vorbeugung oder Behandlung von neurodegenerativen Erkrankungen, wobei die Zusammensetzung Kräuterextrakte aus Genkwae Flos, Clematidis Radix und Gastrodiae Rhizoma enthält,
wobei die Kräuterextrakte 70 %ige Ethanolextrakte sind,
wobei das Mischungsgewichtverhältnis von Genkwae Flos, Clematidis Radix und Gastrodiae Rhizoma 1 : 10 : 10 beträgt und
wobei die neurodegenerative Erkrankung die Parkinson-Krankheit ist.

2. Pharmazeutische Zusammensetzung zur Verwendung bei der Vorbeugung oder Behandlung von neurodegenerativen Erkrankungen nach Anspruch 1, **dadurch gekennzeichnet, dass** der Kräuterextrakt Extrakte aus jeweils Genkwae Flos, Clematidis Radix und Gastrodiae Rhizoma enthält oder aus einem Gemisch aus Genkwae Flos, Clematidis Radix und Gastrodiae Rhizoma extrahiert ist.

3. Pharmazeutische Zusammensetzung zur Verwendung bei der Vorbeugung oder Behandlung von neurodegenerativen Erkrankungen gemäß Anspruch 1, **dadurch gekennzeichnet, dass** die pharmazeutische Zusammensetzung:
(a) Verhaltenseigenschaften verbessert,
(b) durch Toxizität verursachten Zelltod in Nervenzellen hemmt,
(c) die Produktion reaktiver Sauerstoffspezies (ROS) in Nervenzellen hemmt,
(d) die Funktion der Mitochondrien in Nervenzellen schützt,
(e) die Apoptose von Nervenzellen hemmt; oder
(f) die Produktion von Stickstoffmonoxid (NO) hemmt.

## Revendications

1. Préparation pharmaceutique pour une utilisation dans la prévention ou le traitement de maladies neurodégénératives, la préparation contenant des extraits végétaux de bourgeons de fleurs de Daphné lilas, de racines de Clématite, et de rhizomes de Gastrodia,
dans laquelle les extraits végétaux sont composés de 70% d'extraits par éthanol,
dans laquelle le rapport en poids du mélange de bourgeons de fleurs de Daphné lilas, de racines de Clématite, et de rhizomes de Gastrodia est de 1 : 10 : 10, et
dans laquelle ladite maladie neurodégénérative est la maladie de Parkinson.

2. Préparation pharmaceutique pour une utilisation dans la prévention ou le traitement de maladies neurodégénératives selon la revendication 1, **caractérisée en ce que** l'extrait végétal inclut des extraits de chacun parmi les bourgeons de fleurs de Daphné lilas, les racines de Clématite, et les rhizomes de Gastrodia, ou est extrait d'un mélange de bourgeons de fleurs de Daphné lilas, de racines de Clématite, et de rhizomes de Gastrodia.

3. Préparation pharmaceutique pour une utilisation dans la prévention ou le traitement de maladies neurodégénératives selon la revendication 1, **caractérisée en ce que** la préparation pharmaceutique :
(a) améliore la capacité comportementale
(b) inhibe la mort cellulaire causée par la toxicité présente dans les cellules nerveuses
(c) inhibe la production de dérivés réactifs de l'oxygène (DRO) dans les cellules nerveuses
(d) protège la fonction des mitochondries dans les cellules nerveuses
(e) inhibe l'apoptose des cellules nerveuses ; ou
(f) inhibe la production de monoxyde d'azote (NO).
